# FASCICULE DE BREVET EUROPEEN

(11) **EP 3 728 183 B1**
(45) Date de publication et mention de la délivrance du brevet: **14.08.2024**
(21) Numéro de dépôt: 18833926.1
(22) Date de dépôt: 12.12.2018
(51) Int. Cl.: C07C 233/47, A61K 8/34, A61K 8/44, C07C 31/20, A61K 8/64, A61Q 19/00, A61Q 19/10

(54) **NOUVELLE COMPOSITION DE LIPOAMINOACIDES ET D'ALCANEDIOLS, LE PROCÉDÉ POUR LEUR PRÉPARATION ET COMPOSITION COSMÉTIQUE OU PHARMACEUTIQUE EN RÉSULTANT**
NEUE ZUSAMMENSETZUNG AUS LIPOAMINOSÄUREN UND ALKANDIOLEN, VERFAHREN ZUR HERSTELLUNG DAVON UND DARAUS RESULTIERENDE KOSMETISCHE ODER PHARMAZEUTISCHE ZUSAMMENSETZUNG
NEW COMPOSITION OF LIPOAMINO ACIDS AND ALKANEDIOLS, PROCESS FOR THE PREPARATION THEREOF, AND COSMETIC OR PHARMACEUTICAL COMPOSITION RESULTING THEREFROM

(30) Priorité: 20.12.2017 FR 1762622
(43) Date de publication de la demande: 28.10.2020
(73) Titulaire: Société d'Exploitation de Produits pour les Industries Chimiques SEPPIC, 75321 Paris cedex 07 (FR)
(72) Inventeur: GUILBOT, Jérôme, 81100 Castres (FR); DACOSTA, Georges, 81710 Saix (FR); BARTHE, Virginie, 75321 Paris Cedex 07 (FR)
(74) Mandataire: Air Liquide
(86) Numéro de dépôt international: PCT/FR2018/053218
(87) Numéro de publication internationale: WO 2019/122606

(56) Documents cités:
- WO-A2-2013/158824
- FR-A1- 2 765 105
- FR-A1- 2 771 632
- US-A1- 2014 128 309

## Description

La présente invention concerne un nouveau procédé de préparation de dérivés N-acylés d'acides aminés, d'(oligo)peptides ou bien d'hydrolysats partiels ou totaux de protéines, présentant un taux de conversion élevé des matières premières mises en oeuvre.

Les dérivés N-acylés d'acides aminés ou également appelés Lipoaminoacides (LAA), sont des agents tensioactifs anioniques, qui sont constitués par une tête polaire provenant d'un résidu d'au moins un acide aminé, ou bien d'un résidu d'(oligo)-peptides ou bien de résidus d'hydrolysats partiels ou totaux de protéines, et par une chaîne hydrocarbonée de nature lipophile, provenant de chlorures d'acides gras ou d'ester méthyliques d'acides gras, eux-mêmes issus de la filière oléochimique.

Ces dérivés N-acylés d'acides aminés, d'(oligo)-peptides ou bien d'hydrolysats partiels ou totaux de protéines, sont communément utilisés tout d'abord comme ingrédient apportant des propriétés moussantes et nettoyantes pour la préparation de compositions cosmétiques, comme par exemple des gels douche ou des shampoings, ou bien comme ingrédients apportant des propriétés biologiques pour la préparation de compositions cosmétiques destinées à prévenir ou corriger les effets inesthétiques de la peau ; lesdites propriétés biologiques sont par exemple des propriétés anti-âges, amincissantes, raffermissantes, dépigmentantes, propigmentantes.

Les dérivés N-acylés d'acides aminés, d'(oligo)-peptides ou bien d'hydrolysats partiels ou totaux de protéines sont communément synthétisés par acylation d'un ou plusieurs acides aminés en présence de chlorure d'acide, dans les conditions expérimentales dites de Schotten-Baumann.

Un tel procédé est par exemple divulgué dans les brevets américains US 2,463,779 et US 6,703,517, dans la publication J. Am. Oil Chem. Soc.- 78 (1956) 172, et dans les demandes internationales publiées sous les numéros WO 92/21318 et WO 94/26694.

Ce procédé d'acylation comprend une étape préalable de salification de l'acide aminé, suivie d'une étape d'acylation du sel d'aminoacide avec un chlorure d'acide, puis de l'acidification du sel N-acylé obtenu.

La première étape consiste à neutraliser l'acide aminé, solubilisé préalablement dans de l'eau ou dans un mélange d'eau et d'un cosolvant organique, par une base minérale, le plus souvent de la soude ou de la potasse aqueuse. Le groupement carboxyle se retrouve alors sous une forme ionisée, permettant ainsi une meilleure solubilité de l'acide aminé dans l'eau. Le pH de cette solution aqueuse se situe entre 9,0 et 12,0, ce qui permet de s'assurer que la fonction amine de l'acide aminé n'est pas protonée.

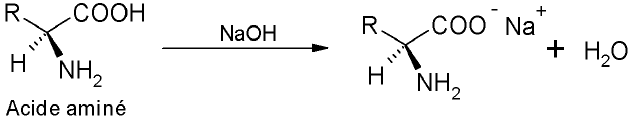

La seconde étape est l'étape d'acylation à proprement dite. A ce stade, le chlorure d'acide est ajouté progressivement à la solution neutralisée d'acide aminé, à température ambiante. La fonction amine nucléophile attaque le carbone électrophile de la fonction carbonyle. Il en résulte la formation d'une liaison amide entre les deux substrats de départ ainsi que la formation d'acide chlorhydrique. Cet acide est directement neutralisé *in situ* par ajout progressif d'une base minérale (régulation du pH autour de 10,0).

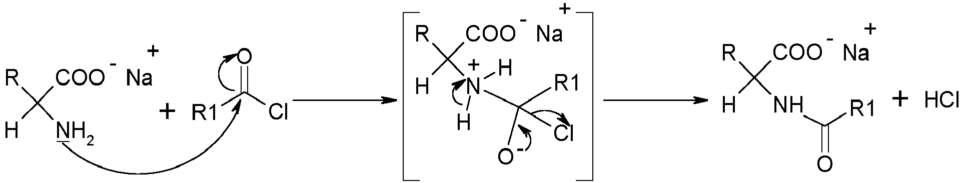

A ce stade, la réaction secondaire d'hydrolyse du chlorure d'acide en savon est également possible. Elle doit cependant être minimisée de façon à atteindre une conversion satisfaisante de l'acide aminé en son dérivé N-acylé et à les isoler efficacement, car une teneur trop élevée en savon (ou sel d'acide gras) peut induire un déphasage du milieu réactionnel et/ou des problèmes d'odeur ou de toxicité (pour des chaînes C₈ et en C'₁₁ par exemple).

Les deux principaux paramètres réactionnels qui permettent de contrôler la formation de savon sont la vitesse d'agitation lors de la phase réactionnelle, dont l'optimisation permet une amélioration de la surface de contact du chlorure avec le milieu, et l'ajout éventuel, lors de l'étape de solubilisation de l'acide aminé, d'un cosolvant d'acylation, comme par exemple l'acétone, la méthyl éthyl cétone, l'isopropanol, ou des glycols.

Cet ajout de cosolvant permet d'améliorer l'affinité du chlorure d'acide avec le milieu réactionnel. Dans un tel cas, le cosolvant d'acylation doit être choisi judicieusement de façon à éviter ou minimiser la formation de nouveaux produits secondaires issus de la réaction entre ce même cosolvant et le chlorure d'acide, comme par exemple des sous-produits provenant de réactions secondaires d'estérification.

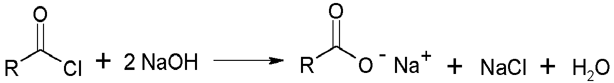

La dernière étape consiste en une étape de finition, de mise en forme du dérivé N-acylé formé, et deux alternatives sont possibles.

La première consiste à régler la valeur du pH du milieu réactionnel obtenu autour de 7. Le dérivé N-acylé est isolé tel quel en solution, sans aucune purification supplémentaire, et comprend les sels d'acylation, les acides aminés non réagis, de l'éventuel cosolvant). Il se présente ainsi sous une forme salifiée, et plus précisément une forme carboxylate, en solution aqueuse et avec une pureté généralement inférieure à 50%. 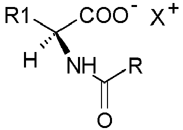
R1 = Chaîne latérale de l'acide aminé,
R = Chaîne cocoyle,
X = Contre-ion.

La seconde consiste à faire précipiter le dérivé N-acylé en acidifiant le mélange réactionnel à une valeur de pH voisine de 2, puis à réaliser plusieurs opérations de filtration et de lavage, se concluant par un séchage final du milieu obtenu. Cette procédure permet ainsi d'éliminer tous les sels générés au cours de la réaction d'acylation, l'éventuel cosolvant d'acylation et tous les acides aminés non réagis. Dans ce cas, le dérivé N-acylé se présente sous une forme non salifiée, avec une fonction acide carboxylique, et une forme solide, plus particulièrement pulvérulente, et avec une pureté supérieure à 80%. 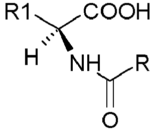
R1 = Chaîne latérale de l'acide aminé,
R = Chaîne cocoyle,
X = Contre-ion.

Le procédé Schotten-Baumann précédemment décrit, présente comme avantage de conduire des réactions de N-acylation avec une cinétique rapide, du fait de la réactivité très élevée des chlorures d'acide vis-à-vis des composés et fonctions nucléophiles (par exemple la fonction amine), sans apport important d'énergie comme par exemple l'énergie thermique, dans un milieu solvant majoritairement constitué d'eau, avec des rendements élevés.

Cependant le formulateur de produits cosmétiques rencontre aujourd'hui des difficultés quant à la mise en oeuvre de poudres, lorsqu'il s'agit de préparer des produits finis.

D'autre part, lorsque des sous-produits provenant de réactions d'estérification secondaires d'estérification sont présents en quantité trop importante, et lorsque le radical acyle de l'acide gras concerné comporte au moins 12 atomes de carbone, on observe une moindre reproductibilité des propriétés biologiques associées aux aminoacides N-acylés formés, et plus particulièrement à la composition comprenant lesdits aminoacides N-acylés ciblés et les produits de réactions secondaires d'estérifications associées. D'autre part, il est communément recherché de limiter la teneur massique en acide gras résiduels inférieure ou égale à 15%, et plus particulièrement la plus faible possible.

C'est la raison pour laquelle il existe un besoin de développer une méthode de synthèse directe de sels d'aminoacides N-acylés salifiés liquides et de haute pureté sans étapes de précipitation / filtration / séchage.

Les inventeurs ont trouvé qu'il était possible d'atteindre ce résultat en utilisant des cosolvants d'acylation pouvant également être utilisés comme solvants finaux de dilution, étant entendu que de tels cosolvants doivent aussi permettre de contrôler et/ou de minimiser la formation d'acides gras lors de l'étape d'acylation, être peu réactifs vis-à-vis des chlorures d'acides dans les conditions de réactions, permettre l'élimination efficace des sels générés au cours de l'acylation par simple décantation liquide/liquide à chaud et enfin permettre d'obtenir une solution d'aminoacides N-acylés homogènes à pH neutre.

La demande de brevet français publiée sous le numéro de publication FR 2765105A2 décrit un procédé de préparation d'un mélange de dérivés N-acylés d'acides aminés mettant notamment en oeuvre une quantité non définie de propylène glycol (ou 1,2-propanediol), du chlorure de lauroyle, et indique un taux d'acide laurique résiduel dans le produit final de 15% massique.

C'est pourquoi, selon un premier aspect, l'invention a pour objet le procédé tel que défini à la revendication 1.

Selon un aspect particulier, l'invention a pour objet le procédé tel que défini précédemment, pour la préparation de la composition (C₁) pour laquelle, dans les formules (I) et (V), X représente le radical méthyle ; selon cet aspect particulier, dans la formule (I), le radical divalent Y représente le radical divalent de formule (IIₐ₁) :

-[NH-CH(R₂)-C(=O)-]ₘ- (IIₐ₁),

dans laquelle m représente un nombre entier supérieur ou égal à un et inférieur ou égal à quatre et R₂ représente un atome d'hydrogène ou un radical choisi parmi les radicaux méthyle, isopropyle, isobutyle, 1-méthyl propyle, benzyle ou 3-amino propyle.

Selon un autre aspect particulier, le procédé tel que défini précédemment, est caractérisé en ce que dans la formule (I), le radical divalent Y représente le radical divalent de formule (II'ₐ₁) :

-NH-CH₂-C(=O)- (II'ₐ₁),

Selon un autre aspect particulier, le procédé tel que défini précédemment, est caractérisé en ce que dans la formule (I), le radical divalent Y représente le radical divalent de formule (II'ₐ₂) :

-NH-CH[-CH(CH₃)-CH₂-CH_{3]}-C(=O)- (II'ₐ₂).

Selon un autre aspect particulier, le procédé tel que défini précédemment, est caractérisé en ce que dans les formules (I) et (V), le radical monovalent X-(CH₂)ₚ-C(=O)- représente le radical palmitoyle.

Les exemples suivants illustrent l'invention sans toutefois la limiter.

### Préparations de compositions (C₁) selon l'invention

### Le mode opératoire communs est le suivant :

➢ Introduction, à température ambiante et sous agitation mécanique, de 20% massique d'isoleucine dans 80% massique d'un mélange Eau/diol (80/20 massique),
➢ Ajout de soude 30% pour atteindre pH ∼ 11,5,
➢ Coulée progressive de chlorure de palmitoyle (0,8 équivalent molaire) en maintenant le pH à -11-12 à l'aide de soude 30%, avec une température de 20 à 40°C.
➢ Cassage à l'acide chlorhydrique 37% qsp pH-2,
➢ Soutirage des eaux mères à 60°C,
➢ Lavage à l'eau saumurée 5%,
➢ Décantation et soutirage à 60°C.

Les caractéristiques des compositions (C₁) sont consignées dans le tableau 1 suivant :

**Table 1**

| | **(C_{1A})** | **(C1B)** | **(C_{1C})** |
|---|---|---|---|
| Diol (IVₐ) ou (IV_{b}) | **Hexylèneglycol** | **1,2-hexanediol** | **1,6-hexanediol** |
| Teneur en diol | 10,2% en masse | 20,1% en masse | 3,9% en masse |
| Teneur en acide palmitoïque (V) | **2,1%** en masse | **2,0%** en masse | 1,3% en masse |
| Teneur en eau | 11,8% en masse | 6,3% en masse | 7,1% en masse |
| Teneur en N-palmitoyl isoleucine (I) | 75,9% en masse | 71,6% en masse | 87,7% en masse |
| Présence de sous-produits | Non | Non | Non |
| pH 1% | 2,7 | 2,3 | 2,5 |

### Préparations d'une composition (C₂₁) comparative

➢ Introduction, à température ambiante et sous agitation mécanique, de 20% massique de Glycocolle (Glycine) dans 80% massique d'un mélange Eau/diol (85/15 massique),
➢ Ajout de soude 30% pour atteindre pH ∼ 10,
➢ Coulée progressive de chlorure de lauroyle (0,9 équivalent molaire) en maintenant le pH à -10 à l'aide de soude 30%, avec une température de 18 à 36°C.
➢ Cassage à l'acide chlorhydrique 37% qsp pH-2,
➢ Soutirage des eaux mères à 60°C,
➢ Lavage à l'eau saumurée 5%,
➢ Décantation et soutirage à 65°C.

Les caractéristiques de la composition (C₂₁) ainsi obtenue, sont consignées dans le tableau 2 ci-après.

### Préparations d'une composition (C₂₂) comparative

➢ Introduction, à température ambiante et sous agitation mécanique, de 20% massique d'un mélange de glycine, d'acide L-aspartique, d'acide L-glutamique et de L-alanine, dans les proportions massiques Glycine/acide L-aspartique/acide L-glutamique/L-alanine, de 10%/35%/45%/10% pour 100% massique dudit mélange d'acides aminés, dans 80% massique d'un mélange Eau/diol (85/15 massique),
➢ Ajout de soude 30% pour atteindre pH - 10,
➢ Coulée progressive de chlorure de lauroyle (0,9 équivalent molaire) en maintenant le pH à -10 à l'aide de soude 30%, avec une température de 18 à 36°C.
➢ Cassage à l'acide chlorhydrique 37% qsp pH-2,
➢ Soutirage des eaux mères à 60°C,
➢ Lavage à l'eau saumurée 5%,
➢ Décantation et soutirage à 65°C.
➢ Les caractéristiques de la composition (C₂₂) ainsi obtenue, sont consignées dans le tableau 2 ci-après.

**Table 2**

| | (C₂₁) | (C₂₂) |
|---|---|---|
| Diol (IVₐ) ou (IV_{b}) | 1,2-propanediol | 1,2-propanediol |
| Teneur en diol (IVₐ) ou (IV_{b}) | 0,9% en masse | 1,9% en masse |
| Teneur en acide laurique (V) | 4,1% en masse | 12,9% en masse |
| Teneur en eau | 11,0% en masse | 27,5% en masse |
| Teneur en N-lauroyl glycine (I) | 83,2 % en masse | 11,9 % en masse |
| Teneur en N-lauroyl alanine (I) | - | 11,0 % en masse |
| Teneur en N-lauroyl acide aspartique (I) | - | 12,2 % en masse |
| Teneur en N-lauroyl acide glutamique (I) | - | 20,4 % en masse |
| Présence de sous-produits | 0,8% en masse | 2,2% en masse |
| pH de la composition | pH = 2,1 | pH = 2,2 |

L'analyse des compositions (C_{1A}), (C_{1B}), et (C_{1C}), objet de la présente invention fait apparaître qu'elles ne contiennent pas de sous-produits et que leur concentration en acide gras est minimale, alors que les compositions comparatives (C₂₁) et (C₂₂), préparées en présence de 1,2-propanediol se caractérisent par une teneur en acides gras résiduels plus élevées et par la présence de sous produits.

## Revendications

1. Procédé de préparation de la composition (C₁) comprenant, pour 100% de sa masse :
**(a)** - Une proportion massique supérieure ou égale à 50% en masse et inférieure ou égal à 95% en masse d'un composé de formule (I) ou d'un mélange de composés de formule (I) :
X-[CH₂]ₚ-C(=O)-Y-OH (I),
dans laquelle , p représente un nombre entier supérieur ou égal 14 et inférieur ou égal à 20, Y représente, soit un radical divalent de formule (IIₐ) :
-[N(R₃)-CH(R₂)-C(=O)-]ₘ- (IIₐ),
dans laquelle R₃ représente l'atome d'hydrogène ou le radical méthyle, m représente un nombre entier supérieur ou égal à 1 et inférieur ou égal à 4 et R₂ représente un atome d'hydrogène ou un radical choisi parmi les radicaux méthyle, isopropyle, isobutyle, 1-méthyl propyle, benzyle, 3-amino propyle ; soit un radical divalent de formule (II_{b}) : 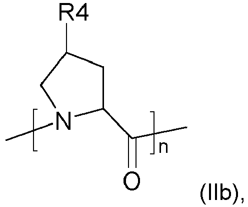 dans laquelle R₄ représente l'atome d'hydrogène ou le radical hydroxyle et n représente un nombre entier supérieur ou égal à 1 et inférieur ou égal à 4 ; et X représente le radical méthyle,
**(b)** - Une proportion massique supérieure à 0% en masse et inférieure ou égale à 25% en masse d'un alcanediol linéaire ou ramifié comportant de six à huit atomes de carbone choisi parmi le 1,6-hexanediol , le 1,2-hexanediol, le 2-méthyl 2,4-pentanediol ou le 1,2-octanediol;
**(c)** - Une proportion massique supérieure ou égale à 0% en masse et inférieure ou égale à 5% en masse d'un composé de formule (V) ou d'un mélange de composés de formule (V) :
X-[CH₂]ₚ-C(=O)-OH (V),
dans laquelle X représente un radical méthyle, p représente un nombre entier supérieur ou égal 14 et inférieur ou égal à 20, ou d'un mélange de dits composés de formule (V) ; et
**(d)** - Une proportion massique supérieure à 0% en masse et inférieure à 50% en masse d'eau,
étant entendu que le pH de ladite composition est inférieur ou égal à 3, **caractérisé en ce qu'**il comprend les étapes successives suivantes :
- Une **étape a)** au cours de laquelle ledit alcanediol comportant de six à huit atomes de carbone, est mélangé avec de l'eau, pour former un mélange (S₁) comportant pour 100% de sa masse, de 5% en masse à 70% en masse dudit alcanediol et de 30% en masse à 95% en masse d'eau ;
- Une **étape b)** au cours de laquelle un composé de formule (VIₐ) : 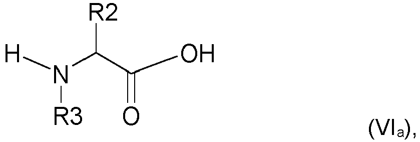 ou de formule (IV_{b}) : 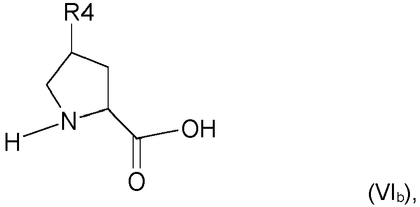 ou un mélange d'au moins un composé de formule (VIₐ) ou (VI_{b}) avec au moins un autre composé de formule (VIₐ) ou (VI_{b}), est ajouté au dit mélange (S₁) en une proportion telle que le mélange (M₁) obtenu comporte pour 100% de sa masse de 5% en masse à 50% en masse dudit composé de formule (VIₐ) ou (VI_{b}) ou dudit mélange de composés de formule (VIₐ) et/ou (VI_{b}) et de 50% en masse à 95% en masse dudit mélange (S₁) ;
- Une **étape c)** au cours de laquelle ledit mélange (M₁) est additionné de soude ou de potasse pour former un mélange (M₂) ayant un pH supérieur ou égal à 9 ;
- Une **étape d)** au cours de laquelle un chlorure d'acide de formule (VII) :
X-[CH₂]ₚ-C(=O)-Cl (VII),
dans laquelle X et p sont tels que définis dans la formule (I) précédente, ou un mélange de chlorures d'acide de formule (VII), est versé dans ledit mélange (M₂), en maintenant le pH à une valeur supérieure ou égale à 9 par addition conjointe de soude ou de potasse, ladite étape e) résultant en la formation d'un mélange (M₃) ;
- Une **étape e)** au cours de laquelle ledit mélange (M₃) est acidifié jusqu'à atteindre une valeur de pH inférieure ou égale à 3 ;
- Une **étape f)** de décantation au fin de soutirage des eaux-mères ;
- si nécessaire ou si désiré, au moins une **étape g)** de lavage à l'eau saumurée pour obtenir après décantation, ladite composition recherchée.

2. Procédé selon la revendication 1, de préparation de la composition (C₁) pour laquelle, dans la formule (I), le radical divalent Y représente le radical divalent de formule (IIₐ₁) :
-[NH-CH(R₂)-C(=O)-]ₘ- (IIₐ₁),
dans laquelle m représente un nombre entier supérieur ou égal à un et inférieur ou égal à quatre et R₂ représente un atome d'hydrogène ou un radical choisi parmi les radicaux méthyle, isopropyle, isobutyle, 1-méthyl propyle, benzyle ou 3-amino propyle.

3. Procédé selon la revendication 2, de préparation de la composition (C₁) pour laquelle, dans la formule (I), le radical divalent Y représente le radical divalent de formule (II'ₐ₁) :
-NH-CH₂-C(=O)- (II'ₐ₁),

4. Procédé selon la revendication 2, de préparation de la composition (C₁) pour laquelle, dans la formule (I), le radical divalent Y représente le radical divalent de formule (II'ₐ₂) :
-NH-CH[-CH(CH₃)-CH₂-CH₃]-C(=O)- (II'ₐ₂),

5. Procédé selon l'une quelconque des revendications 1 à 4, de préparation de la composition (C₁) pour laquelle, dans les formules (I) et (V), le radical monovalent X-(CH₂)ₚ-C(=O)- représente le radical palmitoyle.

## Patentansprüche

1. Verfahren zur Herstellung der Zusammensetzung (C₁), umfassend auf 100 % ihrer Masse:
(a) - einen Massenanteil größer als oder gleich 50 Massen-% und kleiner als oder gleich 95 Massen-% einer Verbindung der Formel (I) oder eines Gemischs von Verbindungen der Formel (I):
**X-[CH₂]ₚ-C(=O)-Y-OH** **(I),**
in der p für eine ganze Zahl größer als oder gleich 14 und kleiner als oder gleich 20 steht, Y entweder für einen zweiwertigen Rest der Formel (IIₐ) steht:
**-[N(R₃)-CH(R₂)-C(=O)-]ₘ-** **(IIₐ),**
in der R₃ für ein Wasserstoffatom oder einen Methylrest steht, m für eine ganze Zahl größer als oder gleich 1 und kleiner als oder gleich 4 steht und R₂ für ein Wasserstoffatom oder einen Rest, der aus Methyl-, Isopropyl-, Isobutyl-, 1-Methylpropyl-, Benzyl- und 3-Aminopropylresten ausgewählt ist, steht; oder für einen zweiwertigen Rest der Formel (II_{b}) steht: 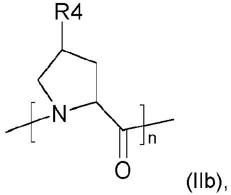 in der R₄ für ein Wasserstoffatom oder einen Hydroxylrest steht und n für eine ganze Zahl größer als oder gleich 1 und kleiner als oder gleich 4 steht; und X für einen Methylrest steht,
(b) - einen Massenanteil größer als 0 Massen-% und kleiner als oder gleich 25 Massen-% eines linearen oder verzweigten Alkandiols mit sechs bis acht Kohlenstoffatomen, ausgewählt aus 1,6-Hexandiol, 1,2-Hexandiol, 2-Methyl-2,4-pentandiol oder 1,2-Octandiol;
(c) - einen Massenanteil größer als oder gleich 0 Massen-% und kleiner als oder gleich 5 Massen-% einer Verbindung der Formel (V) oder eines Gemischs von Verbindungen der Formel (V):
**X-[CH₂]ₚ-C(=O)-OH** **(V),**
in der X für einen Methylrest steht, p für eine ganze Zahl größer als oder gleich 14 und kleiner als oder gleich 20 steht oder, oder eines Gemischs der Verbindungen der Formel (V); und
(d) - einen Massenanteil größer als 0 Massen-% und kleiner als 50 Massen-% Wasser,
wobei es sich versteht, dass der pH-Wert der Zusammensetzung kleiner als oder gleich 3 ist, **dadurch gekennzeichnet, dass** es die folgenden aufeinanderfolgenden Schritte umfasst:
- einen Schritt a), bei dem man das Alkandiol mit sechs bis acht Kohlenstoffatomen mit Wasser mischt, um eine Mischung (S₁) zu bilden, die auf 100 % ihrer Masse 5 Massen-% bis 70 Massen-% des Alkandiols und 30 Massen-% bis 95 Massen-% Wasser umfasst;
- einen Schritt b), bei dem man eine Verbindung der Formel (VIₐ): 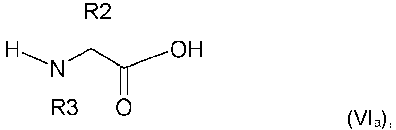 oder der Formel (IV_{b}): 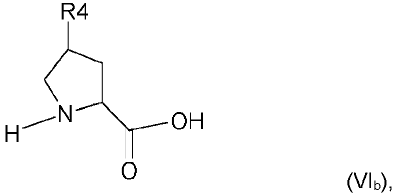 oder ein Gemisch einer Verbindung der Formel (VIₐ) oder (VI_{b}) mit mindestens einer anderen Verbindung der Formel (VIₐ) oder (VI_{b}), in einem solchen Anteil zu der Mischung (S₁) gibt, dass die erhaltene Mischung (M₁) auf 100 % ihrer Masse 5 Massen-% bis 50 Massen-% der Verbindung der Formel (VIₐ) oder (VI_{b}) oder des Gemischs von Verbindungen der Formel (VIₐ) und/oder (VI_{b}) und 50 Massen-% bis 95 Massen-% der Mischung (S₁) umfasst;
- einen Schritt c), bei dem man die Mischung (M₁) mit Natriumhydroxid oder Kaliumhydroxid versetzt, um eine Mischung (M₂) mit einem pH-Wert größer als oder gleich 9 zu bilden;
- einen Schritt d), bei dem man ein Säurechlorid der Formel (VII):
**X-[CH₂]ₚ-C(=O)-Cl** **(VII),**
in der X und p wie in der vorhergehenden Formel (I) definiert sind, oder ein Gemisch von Säurechloriden der Formel (VII) in die Mischung (M₂) gießt, wobei der pH-Wert durch gemeinsame Zugabe von Natriumhydroxid oder Kaliumhydroxid bei einem Wert größer als oder gleich 9 erhalten wird, wobei der Schritt e) zur Bildung einer Mischung (M₃) führt;
- einen Schritt e), bei dem man die Mischung (M₃) bis zum Erreichen eines pH-Werts kleiner als oder gleich 3 ansäuert;
- einen Schritt f) des Dekantierens am Ende des Abziehens der Mutterlaugen;
- falls erforderlich oder falls gewünscht, mindestens einen Schritt g) des Waschens mit Salzwasser, was nach Dekantieren die gewünschte Zusammensetzung ergibt.

2. Verfahren nach Anspruch 1 zur Herstellung der Zusammensetzung (C₁), für die in der Formel (I) der zweiwertige Rest Y für den zweiwertigen Rest der Formel (IIₐ₁) steht:
**-[NH-CH(R₂)-C(=O)-]ₘ-** **(IIₐ₁),**
in der m für eine ganze Zahl größer als oder gleich eins und kleiner als oder gleich vier steht und R₂ für ein Wasserstoffatom oder einen Rest, der aus Methyl-, Isopropyl-, Isobutyl-, 1-Methylpropyl-, Benzyl- und 3-Aminopropylresten ausgewählt ist, steht.

3. Verfahren nach Anspruch 2 zur Herstellung der Zusammensetzung (C₁), für die in der Formel (I) der zweiwertige Rest Y für den zweiwertigen Rest der Formel (II'ₐ₁) steht:
**-NH-CH₂-C(=O)-** **(II'**ₐ₁**)**,

4. Verfahren nach Anspruch 2 zur Herstellung der Zusammensetzung (C₁), für die in der Formel (I) der zweiwertige Rest Y für den zweiwertigen Rest der Formel (II'ₐ₂) steht:
**-NH-CH[-CH(CH₃)-CH₂-CH₃]-C(=O)-** **(II'ₐ₂),**

5. Verfahren nach einem der Ansprüche 1 bis 4 zur Herstellung der Zusammensetzung (C₁), für die in den Formeln (I) und (V) der einwertige Rest X-(CH₂)ₚ-C(=O)-für den Palmitoylrest steht.

## Claims

1. Process for preparing the composition (C₁) comprising, per 100% of its weight:
**(a)** - a weight proportion of greater than or equal to 50% by weight and of less than or equal to 95% by weight of a compound of formula (I) or of a mixture of compounds of formula (I):
**X-[CH₂]ₚ-C(=O)-Y-OH** **(I),**
in which p represents an integer greater than or equal to 14 and less than or equal to 20, Y represents either a divalent radical of formula (IIₐ):
**-[N(R₃)-CH(R₂)-C(=O)-]ₘ-** **(IIₐ),**
in which R₃ represents a hydrogen atom or a methyl radical, m represents an integer greater than or equal to 1 and less than or equal to 4 and R₂ represents a hydrogen atom or a radical chosen from methyl, isopropyl, isobutyl, 1-methylpropyl, benzyl and 3-aminopropyl radicals; or a divalent radical of formula (II_{b}): 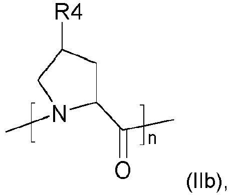 in which R₄ represents a hydrogen atom or a hydroxyl radical and n represents an integer greater than or equal to 1 and less than or equal to 4; and X represents a methyl radical,
**(b)** - a weight proportion of greater than 0% by weight and less than or equal to 25% by weight of a linear or branched alkanediol containing from six to eight carbon atoms chosen from 1,6-hexanediol, 1,2-hexanediol, 2-methyl-2,4-pentanediol or 1,2-octanediol;
**(c)** - a weight proportion of greater than or equal to 0% by weight and of less than or equal to 5% by weight of a compound of formula (V) or of a mixture of compounds of formula (V):
**X-[CH₂]ₚ-C(=O)-OH** **(V),**
in which X represents a methyl radical, p represents an integer greater than or equal to 14 and less than or equal to 20, or a mixture of said compounds of formula (V); and
**(d)** - a weight proportion of greater than 0% by weight and less than 50% by weight of water,
it being understood that the pH of said composition is less than or equal to 3, **characterized in that** it comprises the following successive steps:
- a **step a)** during which said alkanediol containing from six to eight carbon atoms is mixed with water to form a mixture (S₁) containing, per 100% of its weight, from 5% by weight to 70% by weight of said alkanediol and from 30% by weight to 95% by weight of water;
- a **step b)** during which a compound of formula (VIₐ) : 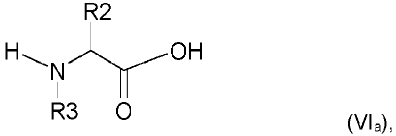 or of formula (IV_{b}): 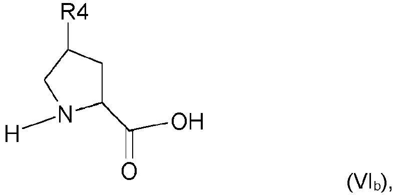 or a mixture of at least one compound of formula (VIₐ) or (VI_{b}) with at least one other compound of formula (VIₐ) or (VI_{b}), is added to said mixture (S₁) in a proportion such that the mixture (M₁) obtained comprises, per 100% of its weight, from 5% by weight to 50% by weight of said compound of formula (VIₐ) or (VI_{b}) or of said mixture of compounds of formula (VIₐ) and/or (VI_{b}) and from 50% by weight to 95% by weight of said mixture (S₁);
- a **step c)** during which sodium hydroxide or potassium hydroxide is added to said mixture (M₁) to form a mixture (M₂) having a pH of greater than or equal to 9;
- a **step d)** during which an acid chloride of formula (VII) :
**X-[CH₂]ₚ-C(=O)-Cl** **(VII),**
in which X and p are as defined in the preceding formula (I), or a mixture of acid chlorides of formula (VII), is poured into said mixture (M₂), while maintaining the pH at a value of greater than or equal to 9 by joint addition of sodium hydroxide or potassium hydroxide, said step e) resulting in the formation of a mixture (M₃);
- a **step e)** during which said mixture (M₃) is acidified until a pH value of less than or equal to 3 is obtained;
- a **step f)** of decantation for the purpose of drawing off the mother liquors;
- if necessary or if desired, a least one **step g)** of washing with brine in order to obtain, after decantation, said desired composition.

2. Process according to Claim 1, for preparing the composition (C₁) for which, in the formula (I), the divalent radical Y represents the divalent radical of formula (IIₐ₁) :
**-[NH-CH(R₂)-C(=O)-]ₘ-** **(IIₐ₁),**
in which m represents an integer greater than or equal to one and less than or equal to four and R₂ represents a hydrogen atom or a radical chosen from methyl, isopropyl, isobutyl, 1-methylpropyl, benzyl or 3-aminopropyl radicals.

3. Process according to Claim 2, for preparing the composition (C₁) for which, in the formula (I), the divalent radical Y represents the divalent radical of formula (II'ₐ₁) :
**-NH-CH₂-C(=O)-** (**II'**ₐ₁),

4. Process according to Claim 2, for preparing the composition (C₁) for which, in the formula (I), the divalent radical Y represents the divalent radical of formula (II'ₐ₂) :
**-NH-CH[-CH(CH₃)-CH₂-CH₃]-C(=O)-** **(II'ₐ₂),**

5. Process according to any one of Claims 1 to 4, for preparing the composition (C₁) for which, in the formulae (I) and (V), the monovalent radical X-(CH₂)ₚ-C(=O)-represents the palmitoyl radical.
